# EUROPEAN PATENT APPLICATION

(11) **EP 1 459 782 A2**
(43) Date of publication of application: **22.09.2004**
(21) Application number: 03022940.5
(22) Date of filing: 09.10.2003
(51) Int. Cl.: A61M 35/00, A61M 37/00

(54) **Method and device for targeted epithelial delivery of medicinal and related agents**

(30) Priority: 21.03.2003 US 394613
(71) Applicant: Hewlett-Packard Development Company, L.P., Houston, TX 77070 (US)
(72) Inventor: Dunfield, John Stephen, Corvallis, OR 97330 (US); Ayres, James W., Corvallis, OR 97330 (US)
(74) Representative: Schoppe, Fritz, Dipl.-Ing.

(57) **Abstract**

A method and device for delivering a quantity of treatment materials to a targeted location on epithelial tissue in which the position on the epithelial tissue is located and a quantity of at least one treatment material is ejected from at least one electronically controllable fluid delivery device into contact with the epithelial tissue.

## Description

### BACKGROUND

The present disclosure pertains to methods and devices for introduction and delivery of materials to a body via epidermal delivery. More specifically, the present disclosure pertains to methods and devices for delivery of materials such as treatment agents to specific targeted regions of epithelial tissue for use thereon or uptake by the body.

Various methods and devices have been proposed for delivering material to epithelial tissue for localized treatment or uptake and remote utilization. Particular materials include, but are not limited to, topical creams, gels and other agents that can be used to treat various skin afflictions for local usage as well as transdermal delivery devices such as medicated patches. Poor or limited control of delivery application can result in harm to the skin in unaffected areas. Thus, various materials which may be useful for transdermal delivery or in the treatment of various topical or localized skin afflictions, such as acne, dermatitis, skin cancers, psoriasis, warts, fungal infections, and the like, can have limited use due to the undesirable effect on normal skin tissue. In certain instances, to mitigate such side effects, the dose concentration of the treatment material must be severely limited.

Treatment of other conditions such as hirsuitism requires the manipulation and/or insertion of mechanical devices into appropriate follicles defined in the epidermal tissue. As with the topical application methods disclosed previously, location of the follicles is, typically, a visual process which is time consuming and prone to error. It would be highly desirable to provide a topical method for the temporary or permanent removal of undesirable hairs, which would be rapid, precise, and eliminate at least some of the invasiveness associated with current hair removal methods.

Transdermal drug delivery methods have also been limited due to the need to use treatment devices such as subcutaneous hypodermic needles or previously prepared transdermal delivery patches. More flexible drug delivery methods which would minimize the need for mechanically invasive epidermal delivery modalities could be desirable.

### SUMMARY

Disclosed herein is a method for delivering a quantity of treatment material to a position on epithelial tissue. The method includes the steps of locating the position on the epithelial tissue and ejecting a quantity of at least one treatment material from an electronically controllable fluid delivery device into contact with the epithelial tissue.

### BRIEF DESCRIPTION OF THE DRAWINGS

Fig. 1 is a process diagram of an embodiment of the method as disclosed;
Fig. 2 is a process diagram of a detailed method as disclosed;
Fig. 3 is a schematic representation of an epithelial delivery device as disclosed;
Fig. 4 is a process diagram detailing actuation and delivery of the method as disclosed;
Fig. 5 is a partial process diagram outlining multiple material delivery;
Fig. 6 is a partial cross section of material applied according to an embodiment of the method as disclosed;
Fig. 7 is a schematic representation of treatment material delivery to a targeted region of epithelial tissue; and
Fig. 8 is a perspective view of a cartridge device suitable for containing treatment material.

### DETAILED DESCRIPTION

Disclosed herein is a method and device for targeted delivery of at least one treatment material to a selected region of epithelial tissue. As used herein, a treatment material can be a permeant, drug, or pharmacologically active agent. The terms "permeant," "drug," and "pharmacologically active agent," are taken to mean any chemical or biological material or compound suitable for epidermal administration by methods previously known in the art and/or by methods taught in the present invention, which would induce a desired biological or pharmacological effect. Such effects may include, but are not limited to:
1) Having a prophylactic effect on the organism and preventing undesired biological effect such as an infection;
2) Alleviating a condition caused by a disease, for example, alleviating pain or inflammation caused as a result of a disease; and/or
3) Either alleviating, reducing, or completely eliminating the disease from the organism.

The effect may be local, such as providing for a local anesthetic effect, or it may be systemic. Such substances include broad classes of compounds normally delivered into the body, including substances delivered through body surfaces and membranes such as the skin or mucosal tissue. In general, this includes, but is not limited to: anti-infectives such as antibiotics and antiviral agents; analgesic and analgesic combinations; anorexics, antihelminthics; antiarthritics, anti-asthmatic agents, anticonvulsants, antidepressants, antidiabetic agents, antidiarrheals antihistamines, antiinflammatory agents, antimigraine preparations, antinauseants, antineoplastics, antiparkinsonian drugs, antipruritics, antipsychotics, antipyretics, antispasmodics, anticholinergics; sympathomimetics, xanthene derivatives, cardiovascular preparations including potassium and calcium channel blockers, beta blockers, alpha blockers, antiarrhythmiacs, antihypertensives, diuretics, antidiuretics, vasodilators including general coronary, peripheral and antihypertensives, diuretics and antidiuretics, vasodilators including general coronary, peripheral and cerebral, central nervous system stimulants, vasoconstrictors, cough and cold preparations including decongestants, hormones such as estradiol and other steroids, including corticosteroids, hypnotics, immunosuppressives, muscle relaxants, parasympatholytics, psychostimulants, sedatives and tranquilizers. It is contemplated that the method of the present invention can be employed to deliver both ionized and non-ionized drugs as well as drugs of either high or low molecular weight. It is also contemplated that the method of the present invention can be employed to deliver microparticals, DNA, RNA, viral agents or any combination of permeants listed above.

As used herein, the term "effective" is defined as a sufficient amount of a compound to provide the desired local or systemic effect and performance at a reasonable benefit/risk ratio attending any medical treatment. An "effective" amount of permeation and chemical enhancer as used herein is defined as an amount selected so as to provide the desired increase in biological membrane permeability, the desired depth of penetration, rate of administration and amount of drug delivered. As used herein, "animal" or "organism" refer to humans or other living organisms having epidermal tissue or its functional analog.

As used herein, the term "tissue" is taken to mean an aggregate of cells of a particular kind, together with their intercellular substance that forms a structural material. At least one surface of the tissue is available for the process of the present invention to be carried out. Typically, the tissue is epithelial tissue as is found in anatomical regions such as the skin, mucosal, and transmucosal tissue. As used herein, "epithelium" and "epithelial" are taken to mean one or more layers of cells covering internal or external structure in an organism. Epithelium may include epidermal cells as typically associated with the skin cells as well as cells covering various internal structures such as those associated with mucosal tissue and the like. "Skin", as the term is used herein is defined as tissue regions characterized by an outer layer or layers of epidermal epithelial tissue, which may be stratified, as well as associated interior layers that may include dermal and transdermal tissue layers. "Mucosal tissue" as the term is used herein is defined as epithelial cells and associated structures found in anatomical regions such as the mouth, nasal passages, and the like. Structures associated with mucosal tissue can include interior layers of epithelial cells, connective tissue, and mucous membranes. "Transmucosal tissue" as that term is used herein includes tissues having epithelial cells and typically found in interior cavity regions and potential spaces such as the digestive and alimentary tracts as well as genitourinary tracts.

As used herein, the terms "poration," "microporation," or similar terms are defined as the formation of a small hole or pore in or through the biological membrane such as skin, mucosal, or transmucosal tissue, or the outer layers of an organism to lessen the barrier properties of this biological membrane to the passage of fluids such as analyte from below the biological membrane for analysis or the passage of active permeants or drugs from without the biological membrane for selected purposes. Preferably, the hole or "micropore" so formed is approximately 1 to 1,000 micrometers in diameter and will extend into the biological membrane sufficiently to break the barrier properties of this layer without adversely affecting the underlying tissue. It is to be understood that the term "micropore" is used in the singular form for simplicity. The device and method disclosed herein may form multiple artificial openings.

The term "penetration enhancement" or "permeation enhancement" is defined as an increase in the permeability of the biological membrane to a drug, analyte or other chemical molecule, compound or particle (also called "permeant') so as to increase the rate at which the drug, analyte or other chemical molecule, compound or particle permeates the biological membrane and facilitates the increase of flux across the biological membrane for the purpose of the delivery of the drugs across the biological membrane and into the underlying tissues.

The terms "enhancer," "chemical enhancer," "permeation enhancer," "penetration enhancer," and the like include all enhancers that increase the flux of a permeant, analyte or other molecule across the biological membrane. These include, but are not limited to cell envelope disordering compounds and solvents as well as any other chemical enhancement agents. Additionally, all active force enhancer technologies such as the application of sonic energy, mechanical suction, pressure, or local deformation of tissues, iontophoresis or electroporation are included. One or more enhancer technologies may be combined sequentially or simultaneously.

It is to be noted that, as used in the specification and the appended claims, the singular forms "a," "an," and "the" include plural referents unless the context clearly dictates otherwise.

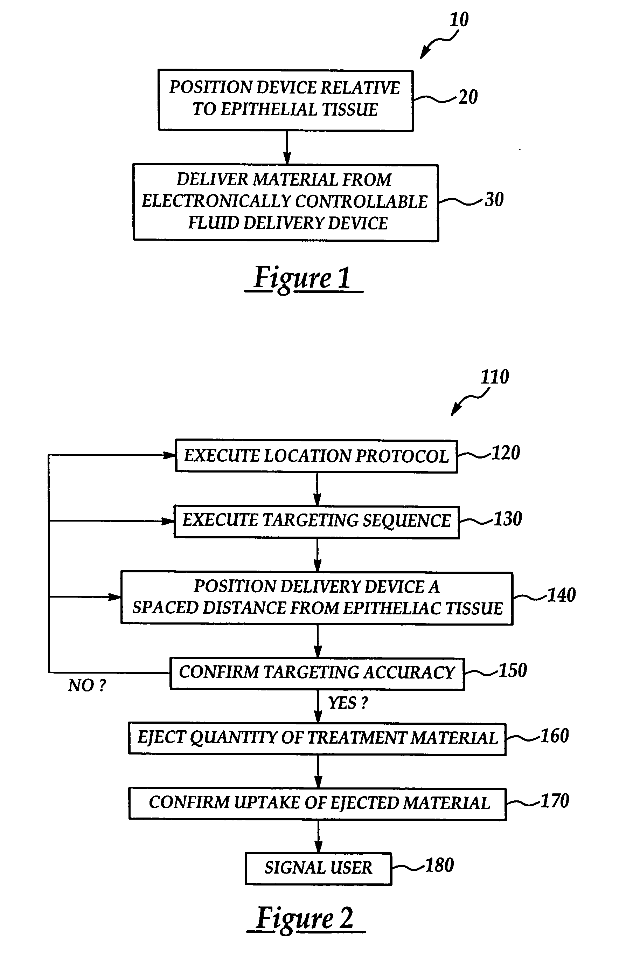

Referring now to Figure 1, a method 10 as disclosed delivers a quantity of a treatment material to a desired position located on the epithelial tissue as at reference numeral 20. Once the position is located, a quantity of at least one treatment material is ejected from an electronically controllable fluid delivery device into contact with the epithelial tissue as at reference numeral 30.

Referring now to Fig 2, method 110 involves the location of the desired position on epithelial tissue such as the epidermis, for example, as at reference numeral 120. Positioning can be accomplished by any suitable process. It is contemplated that a suitable electronically controllable fluid delivery device can be positioned relative to the region of tissue of interest. More specific targeting can occur by appropriate visual or mechanical orientation in which various characteristics of the tissue region are analyzed and quantified. Analysis and quantification can include, but are not limited to, visualization and analysis of variations in tissue topography, visualization and analysis of variations in cell tissue morphology, visualization and analysis of variations in pigmentation and the like. It is contemplated that the variations can be visualized and analyzed by suitable modalities, these modalities include but are not limited to machine vision systems and scanning devices. Data regarding the topography visualized and analyzed by the suitable modality, identified tissue regions, etc., can be integrated into an appropriate location protocol whereby the electronically controllable fluid delivery device is positioned and/or configured to provide optimum delivery accuracy.

Upon execution of the appropriate location protocol, a targeting sequence can be executed as at reference numeral 130. The targeting sequence can include suitable orientation of the fluid delivery device(s) or firing of particular nozzle members within the fluid delivery device(s) to insure precise delivery of the desired treatment material to the optimum position relative to the tissue. "Optimum positioning" as the term is used herein is taken to mean the positioning of the of the electronically controllable fluid delivery device(s) in a manner that achieves the administration of the treatment material central to the region for which treatment is desired or required.

The method as disclosed also includes the positioning of the electronically controllable fluid delivery device a spaced distance from the target position on the epithelial tissue, as at 140. The spaced distance is that sufficient to permit impingement and uptake of the treatment material by at least some of the cells or intercellular matter in the epidermal tissue. Spacing of the electronically controllable fluid delivery device can also be interrelated with the velocity at which the materials are to be ejected from the electronically controllable fluid device into contact with the epithelial tissue, such as the epidermis, mucosal tissue, transmucosal tissue, or the like. Thus, distance and velocity are adjusted to insure uptake of the desired material. The process also contemplates a step in which the targeting accuracy is ascertained and confirmed as at 150. Confirmation of targeting accuracy can be accomplished by analysis of the epithelial tissue in a manner similar to that which occurred during the targeting sequence and location protocol. If targeting accuracy is not confirmed, one or more of the preliminary steps can be reinitiated to achieve desired targeting precision.

Appropriate position and location on the epithelial tissue, such as the epidermis, mucosal tissue, or the like, will depend upon a variety of factors that can include, but are not limited to, the type of disease or condition for which the treatment material is being delivered. Thus, it is contemplated that a localized manifestation such as a wart or the like may require more precise targeting than other types of diseases or conditions. Where the disease or condition includes or is limited to a dermal presentation, it is contemplated that the targeting and subsequent fluid delivery can be in a pattern that corresponds to the atypical morphology or other indicators developed during the location step. Thus, it is contemplated that treatment material can be applied in one or more delivery pulses to directly impinge upon the target cells or to impinge upon the targeted cells and any suitable margin as can be determined by the treatment material and protocol. Where the material administered is directed to disease(s) or condition(s) not specific to epidermal tissue, mucosal tissue, transmucosal tissue, or the like, it is contemplated that targeting and subsequent fluid delivery can be in a pattern appropriate for the particular anatomical region, the atypical morphology, or determined by particular drug delivery requirements or specifications.

The treatment material may be any suitable composition containing one or more compounds which can have an efficacious effect on the epidermal tissue or condition to be treated. In various epithelial tissue treatment situations, for treatment of epidermal tissue, mucosal tissue or the like, the treatment material can include antimicrobial, antibiotic, antifungal, or other pharmacologically active materials that can be delivered from an electronically controllable fluid delivery device into contact with the tissue. Where the material administered is to be utilized for transdermal uptake, the pharmacologically active treatment material may be any compound or agent capable of transdermal uptake or one that can be rendered so by application of suitable ancillary materials.

It is also contemplated that the method can utilize a plurality of electronically controllable fluid delivery devices in contact with various treatment materials which can include, but are not limited to, the antibiotic, antifungal, antimicrobial agents previously mentioned as well as other adjuvants and the like which can be applied in a controllably variable ratio determined by factors which can include, but are not limited to, the nature of the disease or condition under treatment, the necessity of transdermal uptake of previous doses administered, condition of administration site, and the like. It is contemplated that the material or materials will include at least one pharmacologically active agent that can be efficaciously employed in the treatment of the condition detected.

The concentration of the treatment material ejected by the electronically controllable fluid delivery device is that which can be effective in addressing or treating the disease or condition. It is contemplated that targeting accuracy can permit administration of higher concentrations of treatment materials over concentrations employed in other topical application methods to address and treat topical disease(s) or condition(s) without unduly damaging or adversely affecting surrounding tissue. It is also contemplated that concentrations of materials for nontopical diseases or conditions may be advantageously altered given the delivery modality.

Once the position on epithelialtissue such as the epidermis is located, a quantity of treatment material can be ejected from an electronically controllable fluid delivery device into contact with the tissue as at reference numeral 160. The quantity of treatment material(s) ejected from the fluid delivery device(s) will be that suitable for uptake by the targeted region of epithelial tissue.

It is contemplated that the treatment material will be administered as a fluid in droplet form. It is further contemplated that the volume of material that impinges upon the epithelial tissue will be an amount sufficient for uptake and/or treatment of the epithelial tissue. It is contemplated that a degree of volume reduction may occur between ejection from the fluid delivery device and impingement due to evaporation or other phenomena. Thus, the actual volume dispensed may be adjusted to accommodate such volumetric reductions. The quantity of treatment material dispensed will be that which can be taken up by the targeted regions of epithelial tissue to provide the desired tissue response.

The fluid delivery device(s) employed in the method as disclosed provide for electronically controlled generation of droplets and delivery of the generated droplets in a targeted manner over a given distance. The generation and ejection of droplets can be accomplished by suitable mechanisms such as electronically controllable jetting devices having architecture and configurations typically employed in ink-jetting technology. Suitable electronically controllable jetting devices are configured to have electronically controllable nozzle members. Suitable jetting devices can include, but are not limited to, piezoelectric jetting devices, thermal jetting devices, and the like.

Controlled variability in droplet delivery can be governed by suitable control commands that are developed to create the desired administration pattern. Additionally, controlled variability can be governed by calculations that alter or govern the velocity, trajectory, material temperature, and other physical characteristics of the ejected droplet. It is also contemplated that controlled variability can be a function of any or all of the aforementioned considerations. Thus, where desired or required, the temperature of the droplets can be heated to facilitate uptake. Heating would typically occur prior to ejection from the electronically controllable fluid delivery device. Factors such as velocity and trajectory can be controlled to facilitate uptake by increasing mucosal barrier penetration.

The treatment material may be administered in a suitable deposition pattern. The material can be administered as a series of droplets from one or more nozzle members associated with the fluid delivery device such as the jetting device. It is contemplated that multiple droplets can be place in precise location to one another at controlled distances. It is considered within the purview of this disclosure to place ejected droplets to at any distance to within 10 microns of one another if desired or required. Thus ejected material or material can be placed in suitable patterned orientation based on targeting and/or scanning information generated as well as by deposition patterns contained programmed and stored in suitable data storage devices.

The method of material administration may also contemplate an additional step in which the accuracy of delivery of the fluidizable materials is ascertained as at reference numeral 170. Delivery accuracy can be ascertained by any direct or indirect method including visual observation, sensor analysis, as well as dose response observation. In order to further ascertain accuracy of delivery, it is contemplated that the treatment material can include at least one visualization enhancement compound. Suitable visualization enhancement compound(s) can be compounds such as radiological or chemical marker(s) that can be analyzed and detected by a suitable detection mechanism. Accuracy of delivery can include both determination that a material has been brought into contact with the appropriate region of the epithelium such as the epidermis and the ascertainment of locational delivery accuracy. The treatment material may include appropriate affinity markers and the like which would assist in determining that a quantity of fluidizable material was accurately delivered to a desired location on the epithelium.

After the uptake of the ejected material has been confirmed, suitable indicators signaling the user that the material has been successfully administered can be issued as at reference numeral 180. These can include audible and visual indicators of successful administration, which are noted proximate to and or remote from the user.

The method as disclosed can, optionally, include the further step of delivering at least one ancillary material from an electronically controllable fluid delivery device into contact with the epithelium such as epidermal tissue. The compatible material can be delivered at any time prior to, contemporaneous with, or after the delivery of the treatment material. It is contemplated that ancillary material(s) can be those that augment or enhance the action of the treatment material. Such materials can include, but are not limited to, penetrants that facilitate or support the uptake of the treatment material into appropriate cells or intercellular tissue. Uptake facilitators can also include solvents that facilitate permeation of the treatment material through the transdermal barrier for systemic uptake and utilization. Examples of such solvent-based uptake facilitators include, but are not limited to, organic materials such as DMSO (dimethyl sulfoxide).

Pretreatment materials that can be administered can also include materials that minimize uptake of material in regions of the epidermis contiguous to the targeted region. Such materials can function as masks and are impervious to one or more treatment materials to be administered.

It is also contemplated that the ancillary material may be one that can function as an antagonist to the treatment material administered. In an antagonistic response, it is contemplated that the treatment material will be ejected and brought into contact with the tissue. Antagonistic material can be ejected after an interval to limit treatment material action. Alternately, the antagonistic material can be ejected to regions that surround the targeted region to prevent or minimize action of the treatment material in unaffected or undesired regions.

It is contemplated that the antagonistic material can be delivered from associated electronically controllable fluid delivery devices at any suitable time before, during or after ejection of the treatment material, depending upon the nature of the treatment material administered. Thus, it is contemplated that the materials can be administered contemporaneously or in any appropriate sequence. The pattern whereby the antagonistic material is administered can be preset or determined by any suitable analysis that occurs in the targeting process.

Other suitable ancillary materials can include materials that function as adjuvants to the treatment material. Adjuvants can be materials that enhance the action of the administered treatment material and/or enhance its uptake into the appropriate epidermal cells or associated intercellular tissue. Thus, it is contemplated that the material can be a suitable penetrant, binding agent, or the like. The ancillary material can also include material which can function as antimicrobials, astringents, pain killers, analgesics or the like to address or mitigate less desirable side effects, promote healing or other suitable functions.

It is contemplated that the treatment material will be ejected and contact the epithelial tissue at a suitable temperature. Typically, this temperature will be at or above ambient or body temperature. It is also contemplated that the treatment material can be heated prior to delivery from the electronically controllable fluid delivery device where such temperature elevation will enhance the action of the treatment material or its uptake into the cells or intercellular tissue.

The method also contemplates delivery of the treatment material at a velocity and/or trajectory sufficient to penetrate the mucosal boundary defining the epidermal tissue and to facilitate either uptake or transdermal transfer of the delivered material. As necessary, the trajectory and velocity can be varied based upon particular characteristics determined in the cell morphology, tissue topography and the like.

The method of the present invention can also include the step of exerting at least one control limitation on the electronically controllable fluid delivery device such that the treatment material is delivered at the dosage defined by the control limitation. It is contemplated that the control limitation can be derived from one or more factors which can include, but are not limited to, characteristics such as skin condition, dosage schedule, type of treatment material to be administered and the like. If necessary or desired, it is contemplated that the method can also include a step of ascertaining dose response to previously administered doses as a factor in determining the control limitation.

The method as disclosed herein further contemplates the ejection of a second treatment material from an electronically controllable fluid delivery device into contact with epithelial tissue such as the epidermis, mucosal tissue, transmucosal tissue, or the like. The second treatment material is one that differs from the first treatment material and can be one that augments or enhances the first treatment material. It is contemplated that the second treatment material can be ejected simultaneously or sequentially relative to the first treatment material. Thus, it can be appreciated that customized treatment dosages can be delivered to the targeted tissue. Such dosages and ratios can be fixed or can vary depending on factors which can include, but are not limited to, response to previous treatment, further developments in the skin condition and the like. The second treatment material can be targeted for delivery in the same location on the tissue. It is also contemplated that the second treatment material can be delivered to the tissue at a location proximate to the targeted location.

In the method as disclosed herein, at least one treatment material can be delivered from the electronically controllable fluid delivery device into contact with at least one structure defined in epithelial tissue such as the epidermis. Epidermal structures can include transient structures such as localized inflammations such as pimples, blackheads, and the like. It is also contemplated that the structure can be skin tags, various moles, nevuses and the like. Additionally, the present invention contemplates the delivery of material to normal epidermal structures such as hair follicles, pores, and the like. Examples of mucosal tissue structures include, but are not limited to lesions, localized trauma and the like.

Where a treatment material is delivered to an epidermal structure such as a hair follicle, the material may be one that is calculated to effect hair growth from the associated follicle. Thus, the material may be delivered proximate to or delivered directly into the follicle itself. Such materials can be those that may have the effect of stimulating hair growth. Alternately, the administered treatment material may be one that has a depilatory effect of minimizing or eliminating hair growth from the region. The hair removal effect can be temporary or permanent depending upon the nature of the treatment provided. It is also contemplated that targeted delivery of a suitable pharmacologically active material into existing follicles can be utilized to permit subcutaneous uptake of the delivered material. In such situations the morphology and topography of the follicles can be ascertained to optimize targeted delivery of material into the follicle through to the follicle base and into transdermal tissue.

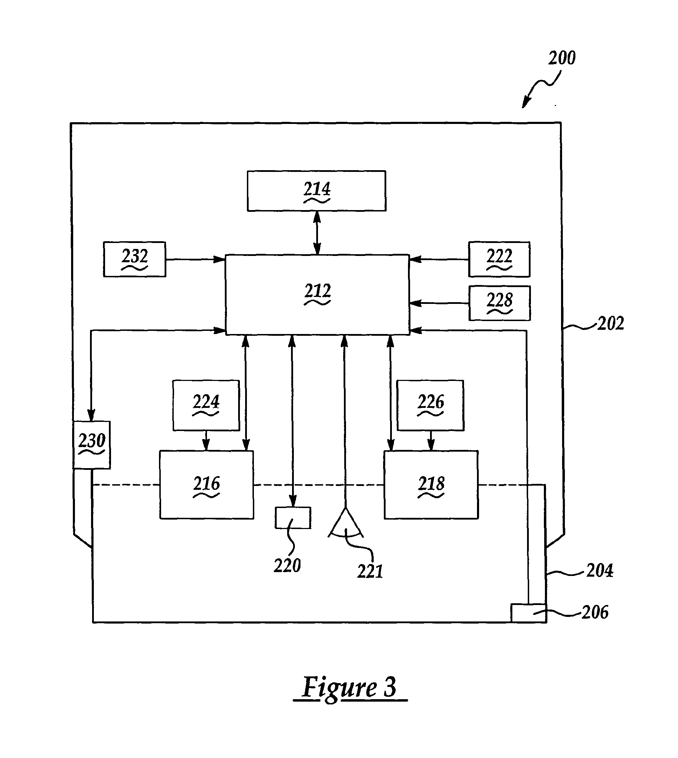

Referring now to Fig. 3, also disclosed is a device 200 for delivering quantities of at least one treatment material into contact with a specified region of epithelial tissue. The device 200 can include a suitable housing 202 that can be configured in any suitable shape or size to facilitate temporary positioning of the device 200 relative to the desired region of tissue. The device 200 can include a suitable spacer configured to permit the lateral positioning of the device and its component parts relative to the desired epidermal tissue. The spacer may be any suitable electronic, mechanical, or electromechanical system for positioning the device 200 relative to the tissue surface or, more specifically, positioning a suitable electronically controllable fluid delivery device 216, 218 relative to the tissue.

As depicted in Fig. 3, the spacer includes a spacer sleeve 204 telescopically received within the housing 202. It is contemplated that the spacer sleeve 204 can be moveably adjusted to provide the appropriate spatial distance between the device 200 and the epithelial tissue. The spacer can also include appropriate devices for interactively and mechanically adjusting the distance between the electronically controllable fluid delivery device and the surface of the epithelial tissue. It is also contemplated that various electronic and electromechanical spacers can be employed. Such spacers include, but are not limited to, systems utilizing electromagnetic field acoustics and systems utilizing various optic and vision systems. As depicted in Fig. 3, the device 200 also can include a contact sensor such as sensor 206 configured to indicate proper contact between the device 200 and the epithelial tissue.

As schematically depicted in Figure 3, the device 200 includes control electronics 212 which are associated or can include an information storage portion or memory 214. The device 200 also includes appropriate electronically controllable fluid delivery devices 216, 218 that are capable of ejecting, delivering, or emitting quantities of an appropriate treatment material or materials. Suitable electronically controllable fluid delivery devices can be those having architecture and configurations found in jetting devices such as those used in inkjets. One or more of the materials delivered may be classified as pharmacologically active depending upon factors which may include, but are not limited, to the treatment regimen, the nature of the epithelial tissue to which the material is to be delivered, the time in a given treatment regimen at which the material is to be administered, and the like.

As depicted in Fig. 3, the device 200 includes an information storage portion 214 associated with control electronics 212. Information storage portion 214 is configured to contain and/or receive information relevant to various aspects of the administration of the material or materials. These aspects can include the quantity of material or materials delivered with each activation of the device 200. It is contemplated that the quantity or quantities of the given materials can programmably vary relative to one another. Variation of the quantities of materials can occur over time, at given intervals, or can occur after a desired number of activations of the device. It is also contemplated that variation of the quantity of material can occur based upon analysis of the epithelial tissue to which the material is to be delivered.

It is also contemplated that the targeting or delivery pattern may be altered based upon the factors previously enumerated. Thus, it is contemplated that the pattern of administration of material ejected from the electronically controllable fluid delivery devices 216, 218 can be varied to permit specified delivery of the materials as desired or required.

Control electronics 212 may be any configuration of hardware and/or software that can maintain logic and circuitry capable of interactive function with the electronically controllable jetting devices 216, 218 employed in device 200. As depicted if Fig. 3, it is contemplated that suitable control electronics 212 can be capable of interactive communication and control with associated electronically controllable fluid delivery devices 216, 218 as well as receiving input from various other sources and devices which can include, but are not limited to targeting site mechanisms 220 and user interface 222.

Information storage may occur in the information storage portion 214. Pertinent information can include, but is not limited to, data regarding dosing instructions, drug interactions, dosing interval, tissue morphology, identification and the like. It is contemplated that such information may be preprogrammed into the information storage portion 214 prior to initial user activation. It is also considered within the purview of this invention that the information storage portion 214 may be configured to receive command instruction at any point during the use and cycle of the device 200. Thus, in certain embodiments, it is contemplated that the information storage portion 214 may be configured to receive various operational instructions from external medical personnel and the like. Such operation instructions may augment basic programming and dosage administration information. It is contemplated that the device 200 can include a suitable material such as interface 222 to permit receipt of operation instructions and/or download of information contained in the device. Examples of such interfaces include, but are not limited to, infrared communication links, physical communication links, touch pads and the like. It is contemplated that the device 200 as disclosed may be configured with appropriate hardware and software to accomplish web-enabled communication, wireless enabled communication, or other suitable one-way or two-way communication strategies or protocols. Where desired or required, the device may be configured with appropriate docking or linking capability to permit or facilitate linked communication. Examples of such capabilities include, but are not limited to, configurations employed with PDA's. Such systems are generally employed to facilitate the secure transfer of data from the device 200 to one from a remote source.

Furthermore, it is contemplated that the information storage portion 214 may be employed to provide analytic and decision-making capability based upon observation and data derived from targeting device 220 or from other sensors or imaging systems such as imaging system 221. Imaging system 221 may be any optic or digital scanning system or combination thereof capable of discerning at least one characteristic incident to targeting, topography, location, or tissue identification. Examples of such imaging systems are systems capable of scanning and recognizing images and characteristics. These can include, but are not limited to, optic scanning systems, acoustic recognition systems, and photosensor systems. It is also contemplated that the imaging system may be configured to detect single or multiple components either present or administered to the epidermal region.

The imaging system 221 may also include suitable measurement and/or vision devices which can discern at least one of the distances between the device 200 and the surface of the epithelial tissue, variations or deviations in cellular tissue structure in the epithelial tissue, and topographic variations on the epidermal tissue of interest. The imaging system 221 may be equipped with suitable detection systems that can detect variations in cell morphology and plot relative position of specific cells within a given targeting field either independently or in communication with control electronics. As depicted herein the imaging system can be in electronic communication with control electronics 212 and information storage portion 214 to produce interactive communication and control of elements such as electronically controllable fluid delivery device 216 and targeting device 220. In order to identify specific cells or tissue regions, it is contemplated that the imaging system 221 can be configured to identify tagging components introduced into the tissue region. Tagging components may be those capable of selective uptake or association with specific cells within the epithelial region. Selective uptake can be due to characteristics in cell morphology and/or activity. It is contemplated that a detectable tagging component suitable for preferential uptake by specific cells or regions may be applied by any appropriate application method. Such application methods can include but are not limited to, systemic injection into the organism and preferential uptake, topical injection and infusion, and topical application. Where topical application is employed, it is contemplated that the detectable compound may be applied by a separate applicator or may be administered by suitable electronically controllable fluid delivery device(s) such as fluid delivery devices 216, 218.

It is also contemplated that the imaging system 221 can have capability to validate appropriate delivery of a treatment material or materials. Validation can be accomplished by suitable observation and scanning by imaging system 221 and may be enhanced by incorporating suitable tracer materials detectable by the imaging system into the treatment material. Detected material can be visualized by a suitable digital or analog device. Signal data received can be processed to confirm accuracy of the delivery.

It is contemplated that imaging system 221 and targeting device 220 can function to interactively identify epithelial tissue regions for administration of materials. The imaging system 221 can gather and transmit data suitable to derive delivery parameters for appropriate administration and uptake of the material(s). Delivery parameters can include, but are not limited to, space between delivery device and tissue, velocity of material delivered, evaporation rate of material during delivery, and temperature of material. Targeting information such as delivery parameters can be maintained in suitable information storage devices such as the information portion 214 of control electronics 212.

Information such as delivery parameters as well as other device control protocols may be contained in information storage in any manner that permits conversion to appropriate control signals. Thus stored information need not be directly readable from device 200.

Targeting device 220 can act to position key components such as electronically controllable fluid delivery devices 216, 218 to provide appropriate spacing between an electronically controllable fluid delivery device and epithelial tissue.

Adjustment of the spacing between the electronically controllable fluid delivery devices 216, 218 and the epidermal tissue can be achieved by various mechanisms and routines. For example, as depicted in Fig. 3, the device 220 can include a suitable adjustment mechanism 230 attached to the housing 202 and is configured to telescopically position spacer sleeve 204 relative to housing 202. Spacer sleeve 204 has a sensor 206 located proximate to its distal edge adapted to confirm position relative to or contact with epidermal tissue. Telescopic movement of the spacer sleeve 204 relative to the housing 202 can be employed to position the associated electronically controllable fluid delivery 216, 218 at the desired spaced distance from the epidermal tissue to permit appropriate delivery of the ejected treatment material to the epithelial tissue.

The targeting device 220 and imaging system 221 may function interactively to provide suitable mapping capacity to permit the resolution of a suitable targeting field that will be contacted by one or more treatment materials. It is contemplated that the precise targeting field as mapped can be dosed by the programmed firing of selected nozzle members present on one or more associated electronically fluid delivery device 216, 218 in a controlled manner developed by the control electronics 212 and associated information storage portion 214. Thus the device 200 can lay down a patterned deposition of one or more treatment materials based upon the information developed.

The device 200 includes electronically controllable fluid delivery devices 216, 218. While first and second electronically controllable fluid delivery devices 216, 218 are specifically depicted and discussed, it is to be understood that the device 200 may include any number of fluid delivery devices desired or required to administer various treatment material into contact with the targeted epithelial tissue region.

The electronically controllable fluid delivery devices 216, 218 may be suitable microfluidic devices capable of producing or emitting material in a volumetric size range and velocity appropriate to facilitate introduction and uptake of the treatment material(s) into the epidermal tissue. Suitable electronically controllable fluid delivery devices may incorporate control and structural features commonly associated with inkjet printing devices. Such devices can include, but are not limited to, piezoelectric devices, thermal fluid jetting devices, vibrating membrane devices with piezoelectric actuators and the like which are capable of dispensing material in droplet form upon receipt of an appropriate activation command.

The electronically controllable fluid delivery devices 216, 218 may be fluidically coupled to any suitable source of treatment material as desired or required. Treatment material sources can be either remote or proximate to the respective electronically controllable fluid delivery device 216, 218. The reservoirs 224, 226 may be configured to facilitate on-axis or off-axis delivery of treatment material to the associated electronically controllable fluid delivery device(s) 216, 218. As depicted in Fig. 3, suitable materials are maintained in reservoirs 224, 226 located in device 200 in fluid communication with the associated electronically controllable fluid delivery device 216, 218.

The device 200 may also include a suitable actuator. The actuator 228 may be a suitable trigger operated by the user to initiate dose dispensation. The actuator 228 may be coupled with a suitable external sensor, on/or switch or other appropriate mechanism to permit actuation of the device 200 as desired or required. The actuator may be associated with user interface 228 or may be a separate element as desired or required.

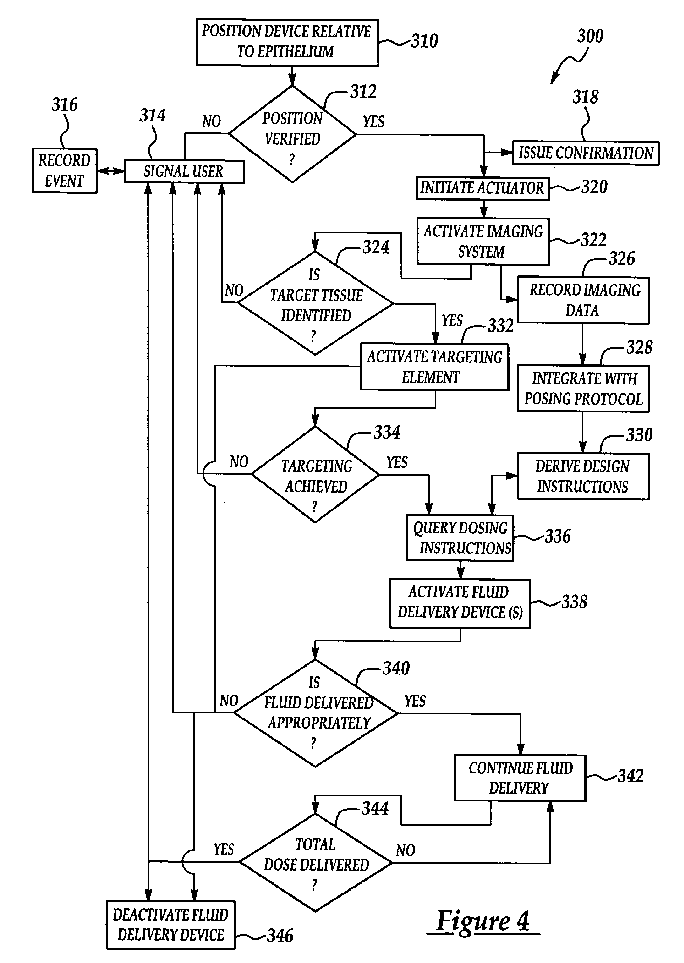

Actuation may include any suitable sequence that culminates in the delivery of treatment material to a targeted location on the epidermal tissue. A basic actuation sequence is outlined in Fig. 4. In the basic actuation sequence 300, the device 200 is positioned relative to epidermal tissue as at reference number 310. Positioning of the device 200 relative to epithelial tissue such as the epidermis, mucosal tissue, transmucosal tissue, or the like, can be verified as at reference numeral 312. Position verification can be accomplished by any suitable means such as by data received from a suitable thermal device, touch sensor, or the like. Failure to obtain position verification can be signaled to the user in any suitable manner such as by an audible or visual signaling device as at reference numeral 314. Additionally, the failure can be recorded as at reference numeral 316 if desired or required. It is contemplated that position verification can be utilized to prevent operation or misfiring of device 200.

Position verification can be accompanied by a suitable confirmation signal as at reference numeral 318. Positive confirmation of position verification can be followed by initiation of actuator as at reference numeral 320. Actuator initiation can be an automatic step following position verification as depicted in the process diagram of Fig. 4. Alternately, actuator initiation can proceed upon receipt of an externally originated command as from the user or other source(s). An example of an externally received command would be one received as a result of user activation of actuator 228, which may include a suitable touch sensor, an on/off switch, or the like.

Initiation of actuator as at reference numeral 320 signals activation of imaging system as at reference numeral 322. The imaging system may provide suitable visual, thermal, topographic, or other scanning data required to map and/or identify epithelial tissue in the region of interest. More particularly, the imaging system can function to identify epithelial tissue targeted for treatment material delivery and/or treatment as at decision junction 324.

Imaging data can be recorded as at reference numeral 326. Recorded imaging data can be retained for future reference. It is also contemplated that relevant portions of the imaging data can be integrated with dosing protocol or parameters as at reference numeral 328 to derive dosing or administration instructions as at reference numeral 330. Imaging data can include, but is not limited to, information pertaining to topography and/or tissue morphology that can be relevant to identifying regions requiring treatment or dosing relative to regions that do not. Integration of such information with dosing protocol or parameters can permit generation of customized dosing instructions that can include customized mapping of materials) to be administered. It is contemplated that dosing protocols or parameters may be maintained in appropriate information storage elements such as the information storage portion 214 or may be received from appropriate sources external to the device 200 as through interface 222.

Failure to identify targeted tissue can result in a user signal such as at reference numeral 314. Positive identification can result in a signal or command that initiates activation of targeting element as at reference numeral 332. Targeting proceeds to insure proper spacing of delivery devices from the tissue regions of interest.

Failure to achieve targeting as at decision junction 334 can result in a suitable user signal and event recordation. Positive indication that targeting has been achieved permits a query for and retrieval of dosing instructions as at reference numeral 336. As depicted in the process diagram set forth in Fig. 4, dosing instructions can be derived from protocols integrated with imaging data. Alternately, the dosing instructions may be obtained from a standard library maintained in suitable on-board information storage or in suitable external sources.

As used herein, "targeting" can include various adjustments in elements in the device 200 to achieve accurate delivery of the material(s). Adjustment can include, but is not limited to, at least one of adjustment of the distance between the electronically controllable fluid delivery device(s) and the targeted epidermal tissue region of interest, adjustment of the ejection velocity of the quantities of the material(s) dispensed from the electronically controllable fluid delivery device(s), adjustment of the temperature of the materials) upon ejection, and adjustment in droplet size and trajectory.

As used herein, "dosing instructions" can include the amount and concentration of any material(s) to be dispensed as well as the pattern and sequence in which the material(s) are to be dispensed. Application of a treatment material or materials can be accomplished by the actuation of fluid dispensing device(s) as at reference 338. Actuation can occur in a manner to achieve a dispensing pattern appropriate to accomplish the targeted application of the material(s) desired. It is also contemplated that a given application event may involve one or multiple activations of a suitable electronically controllable fluid delivery device. Thus, where treatment material can be taken up by the epithelial tissue in a single dosage application, the material can be applied in a single activation event. Where uptake is facilitated by rapid multiple activation events, the electronically controllable fluid delivery device(s) can be activated to accomplish such sequence.

The process as outlined in Fig. 4 also contemplates confirmation of delivery as at decision junction 340. Confirmation can be accomplished by a suitable scanning or analysis sequence. Nonlimiting examples of such confirmation sequences include inferential analysis of material administered through the electronically controllable fluid delivery device(s) and analysis of the epidermal tissue to confirm delivery and physical uptake using various associated analytical sensors and imaging devices.

Positive indication of fluid delivery results in continued delivery as at reference numeral 342. Negative indication results in a user signal as at reference numeral 314 and/or adjustment of the delivery sequence.

Fluid material delivery continues until indication is received that total dose has been delivered as at decision junction 344. Positive indication of total dose delivery results in deactivation of fluid delivery device as at reference numeral 346 and user signal as at 314. The event can be recorded together with dosage and relevant observational data.

Total delivery can be monitored by any suitable device analyzing any number of physical characteristics that can include at least one of delivery volume, delivery interval, observed dose response, and the like. Observed dose response can include at least one of physical changes in the treated area on a macro or micro scale and observed uptake of material administered.

It is contemplated that various different materials can be applied using the device 200 as disclosed herein. As indicated, various materials can include various treatment materials having pharmacological activity as well as those having protective, analgesic, antagonistic, restorative and/or palliative effects. It is contemplated that a primary treatment material may be administered in sequence or simultaneously with other ancillary materials as desired or required.

It is further contemplated that the application pattern for ancillary materials may be essentially the same as the application of the primary material or may be complimentary to the application pattern for any primary material administered. It is also contemplated that the ancillary materials may be administered by a separately derived targeting program that is based on a suitable factor which can include, but is not limited to cell morphology, tissue topography, and the like. Depending upon the condition to be treated, the primary treatment material may be one that is precisely applied to the epithelial region of interest. Precision application of the primary treatment material to a narrowly defined affected region using the device 200 may permit application of treatment material that is more concentrated or may have heretofore been unfavored for topical application due to an undesirable side effects panel manifested upon application to surrounding epithelial tissue. By way of nonlimiting example, the primary treatment material administered into contact with the epidermal tissue may be a pharmaceutical compound exhibiting oncological activity on cancerous and pre-cancerous epidermal cells. Targeted application to identified cancerous and pre-cancerous cells can arrest undesirable cell growth while minimizing or eliminating adverse effect on surrounding healthy epidermal tissue. The device 200 may also be employed to introduce antagonistic agents to surrounding healthy epidermal tissue to further mitigate adverse effects. Such introduction can be contemporaneous with the introduction of the primary treatment material or may be sequentially administered before or after application of the primary treatment material as desired or required. It is also considered within the purview of the method disclosed to apply an antagonist or other mask material independent of application of other treatment materials.

It is also possible to employ the device 200 to administer primary treatment material(s) in combination or sequence with local anesthetics, local analgesics, or various palliative or protective agents which can minimize adverse effects caused by the administration of the primary treatment material(s) or can enhance action by the primary treatment materials. The additional material may be administered in a targeted manner to the region of interest, a region complimentary to the region of interest, or to a region independently identified and targeted by the device 200 or by other means.

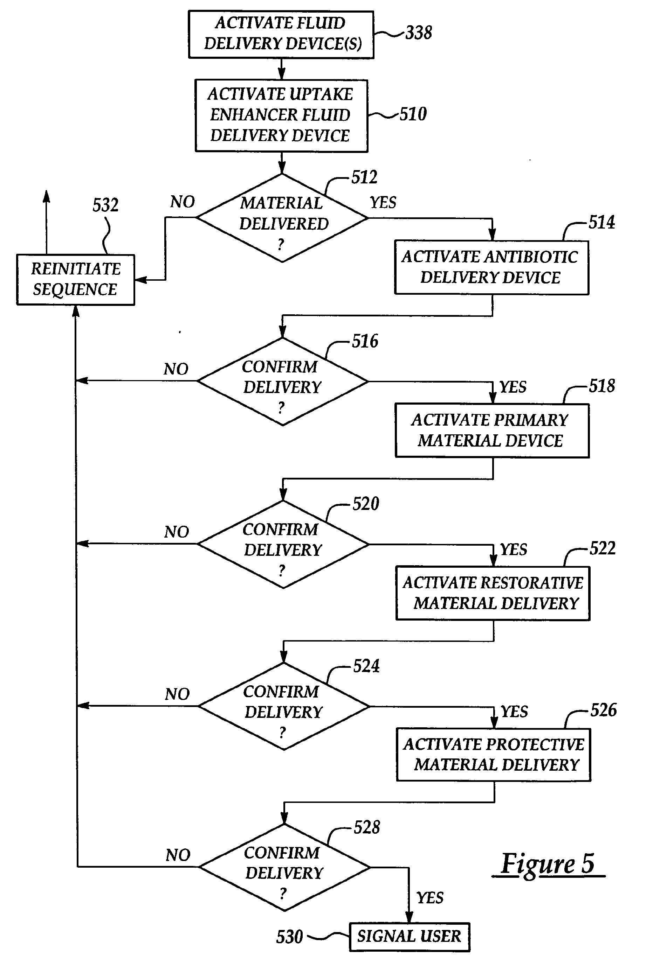

Referring now to Fig. 5, there is outlined a detailed fluid delivery device activation sequence 338 for providing multiple materials and an overlayment layer. It is contemplated that such sequences could be advantageously employed to treat various epidermal conditions or trauma and provide a protective, potentially bacterial resistant, overlayment layer once treatment materials have been delivered.

Activation of fluid delivery devices as at reference numeral 338 can begin sequential and/or concurrent activation of multiple fluid delivery devices according to an exemplary dosing regimen such as derived dosing instructions 330. As depicted in Fig. 5, fluid delivery device(s) capable of delivering an uptake enhancer fluid can be activated initially as at reference numeral 510. As used herein uptake enhancement is defined as a process whereby cells and intercellular material are prepared for facilitated uptake of treatment materials. Examples of uptake enhancers include, but are not limited to, biologically compatible surfactants and solvents.

As depicted in Fig. 5, once delivery of the enhancer fluid has been confirmed as at decision junction 512, an electronically controllable fluid delivery device(s) administering pretreatment materials like antibiotics, antifungals, and the like can be administered as at reference numeral 514. Suitable antibiotic or antifungal materials can be either systemic or localized depending upon the nature of the condition being treated. Additional ancillary materials can be administered as desired or required. Such materials include, but are not limited to, materials such as masks, antagonists and the like.

Once delivery of the material(s) has been confirmed, as at decision junction 516, a primary material or materials can be administered as at reference numeral 518. Once delivery of the primary treatment material has been confirmed as at reference numeral 520, additional post-treatment materials can be administered such as restoration enhancement materials that facilitate cell growth or recovery as at reference numeral 522. Other materials which could be administered at this point could include analgesics, palliatives, and local pain medications to address any lingering pain experienced as a result of the treatment procedure or unrelated trauma.

Once delivery of the restorative material has been confirmed as at decision junction 524, suitable electronically controllable fluid delivery devices can be activated to administer at least one layer of protective material to overlie the targeted region in a manner which protects and promotes healing as at reference numeral 526.

The protective material can be a suitable material that covers the epidermal region in a permanent or semipermanent fashion to minimize infiltration of air, water, contaminants and the like. It is contemplated that "permanent" material is of a type that would be physically removed by application of a peeling or prying force. "Semipermanent" materials are considered those that would degrade over time. The material may be a binder-type material that is non-interactive or inert to epidermal uptake or interaction.

The protective material may be transparent or pigmented as desired or required. The protective material may also include suitable tracers or marking compounds or features to indicate treatment location and or type. Depending upon application thickness and the nature of the material applied, it is also contemplated that the material may contribute to the structural support of the underlying epidermal tissue to maintain tissue position, etc.

Once application of the protective material has been confirmed as at decision junction 528, a suitable user signal can be generated indicating that treatment has been successfully completed as at reference numeral 530.

As outlined in Fig. 5, it is contemplated that a negative indication of material delivery at any decision junction 512, 516, 520, 524, 528 can result in appropriate reinitiation of the application sequence as at reference numeral 532 to provide material delivery.

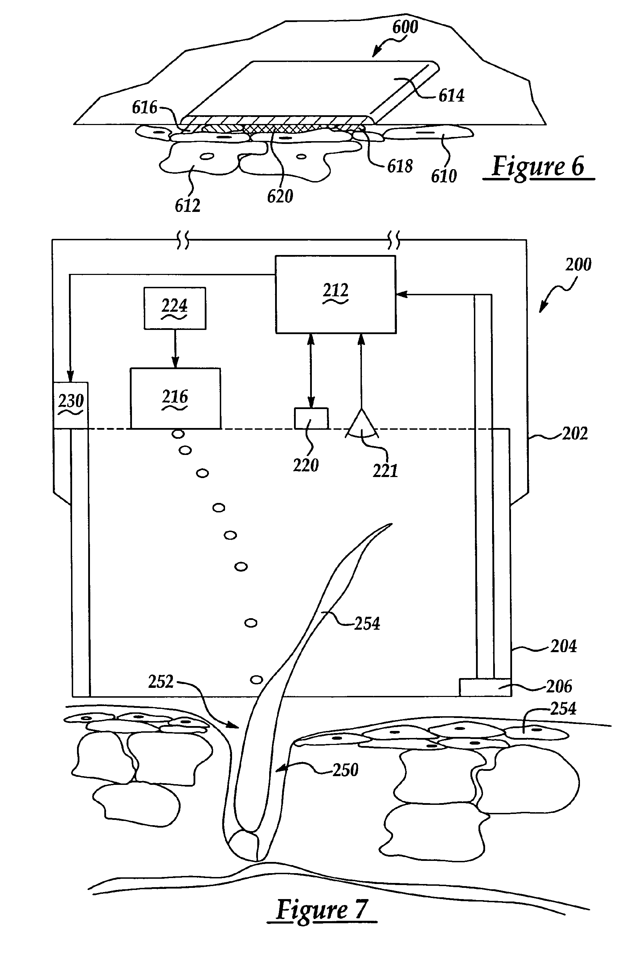

As depicted in Fig. 6, the process as depicted in Fig. 5 can be employed to provide an epidermal patch 600. The epidermal patch may be employed to cover regions treated by fast-acting and/or fast-uptake materials applied to epidermal tissue 610 and underlying layers 612. The patch 600 can include an outer layer or layers 614 formed of a protective barrier or binder material having an inwardly oriented surface 616 overlying one or more intermediate layers 618, 620. The inner surface 616 of the protective barrier layer 614 and intermediate layers 618, 620 are adapted to conform to the outer surface of the epidermal layer and to one another. Thus the inner surface 616 of barrier layer 614 can maintain slower acting intermediate materials in position relative to the epidermal tissue. It is also contemplated that the protective barrier or binder layer 614 can be configured to contain treatment materials that are capable of migration through the barrier layer 614 for treatment of epidermal tissue or transdermal absorption. Materials suitable for use in the barrier or binder layer are those that will provide a protective surface and/or are essentially inert to epidermal uptake.

Referring now to Fig. 7, a device 200 is utilized to administer depilatory agents in a targeted deliverable manner to a follicle 250 or the epidermal region immediately surrounding the follicle. In utilizing the device 200 to deliver a material such as a hair removal agent, the epidermal tissue 254 can be scanned by targeting mechanism 220 and imaging system 221 to identify suitable hair follicle(s) 250 and to target the electronically controllable fluid delivery device(s) 216 to administer treatment material into the region defined at the follicular opening. The treatment material can be material formulated to remove the hair shaft 254 emanated from the follicle 250 to achieve an effective targeted depilatory action. The treatment material can be further formulated to retard or eliminate additional hair growth. It is also contemplated that the device 200 can include additional jetting devices that can emit materials formulated to condition the surrounding epidermal tissue 254 and/or to provide analgesic, antimicrobial and/or anesthetic effects to the surrounding tissue.

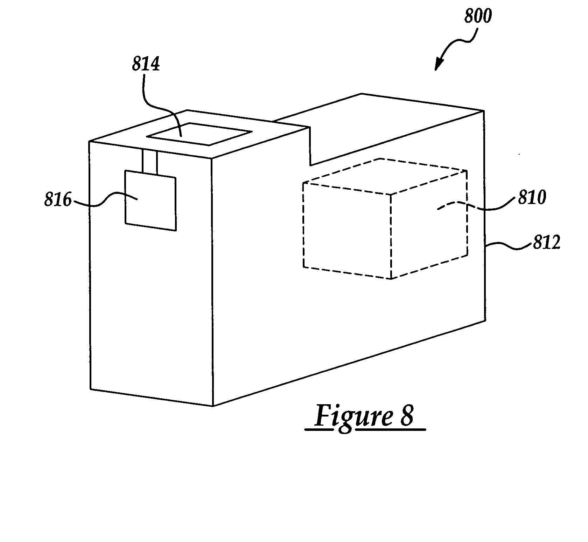

It is contemplated that the device 200 as disclosed can be employed as a single use or multiple use item. It is also contemplated that the device 200 can be configured to be refillable if desired or required. It is also contemplated that the device 200 may be a unit utilizing replaceable cartridges containing one or more treatment materials as depicted in Fig. 8. The cartridge 800 can include a housing 810 with at least one reservoir located in the housing, which can contain at least one treatment material as well as any other compatible materials desired or required. The cartridge 800 includes electronically controllable fluid delivery devices such as device 814 associated with the housing 810 in fluid communication with the reservoir. The electronically controllable fluid delivery device(s) dispense material from the associated reservoir 812 such as suitable treatment material toward the epidermis. The device 800 can also include a suitable memory element 816. It is contemplated that the memory element 816 present on the cartridge 800 can function interactively with suitable counterparts on an independent device to delivery material in a targeted manner to the desired location on the epidermis.

While the invention has been described in connection with what is presently considered to be the most practical and preferred embodiment, it is to be understood that the invention is not limited to the disclosed embodiments but, on the contrary, is intended to cover various modifications and equivalent arrangements included within the spirit and scope of the appended claims, which scope is to be accorded the broadest interpretation so as to encompass all such modifications and equivalent structures as permitted under the law.

## Claims

1. A method (110) for delivering treatment material to epithelial tissue, the epithelial tissue including at least one of epidermal tissue, mucosal tissue and transmucosal tissue, the method comprising the steps of:
scanning a region of epithelial tissue to obtain scanning data;
locating (120) a position on the scanned region of epithelial tissue; and
ejecting (160) a quantity of at least one treatment material from an electronically controllable fluid delivery device into contact with cells associated with the scanned region of epithelial tissue.

2. The method of claim 1 further comprising the step of positioning (140) the electronically controllable fluid delivery device a spaced distance from the epithelial tissue, the distance sufficient to permit impingement and uptake of the treatment material by at least some cells in the epithelial tissue

3. The method of claim 1 or 2 wherein the treatment material is ejected at least one of a velocity and a trajectory sufficient to position the treatment material at a location on the epidermal tissue which penetrates a mucosal barrier defining the epithelial tissue.

4. The method of claim 1, 2 or 3 further comprising the step of exerting a control limitation on the electronically controllable fluid delivery device wherein the treatment material is ejected from the fluid delivery device at a dosage defined by a control limitation, the control limitation including at least one skin condition, dosage schedule and type of treatment material.

5. The method of claim 1, 2, 3 or 4 wherein the quantity of the treatment material is delivered over a targeted area, the concentration of treatment material delivered variable with regard to position in the targeted area.

6. The method of claim 1, 2, 3, 4 or 5 wherein the treatment material is ejected from the electronically controllable fluid delivery device into contact with at least one structure defined in the epithelial tissue.

7. The method of claim 1,2, 3, 4, 5 or 6 further comprising the step ejecting a quantity of a second treatment material from an electronically controllable fluid delivery device into contact with the epithelial tissue wherein the second treatment material differs from the first treatment material.

8. A device (200) for delivering a quantity of at least one treatment material to a defined location of epithelial tissue (254), the device comprising:
an electronically controllable fluid delivery device (216, 218) in fluid communication with at least one treatment material (224, 226);
a spacer (204) adapted to a position the electronically controllable fluid delivery device a spaced distance from an anatomical region which includes the epithelial tissue (254); and
control electronics (212) in communication with the electronically controllable fluid delivery device and the spacer, the control electronics having an information portion (214) which includes an information for delivering a quantity of at least one treatment material into contact with the epithelial tissue, the information including at least one of total dosage, programmed dispersing regimen, interval since last dispensation, and use frequency.

9. The device of claim 8 further comprising a targeting array (220), the targeting array capable of orienting the electronically controllable fluid delivery device (216, 218) relative to a position on the epithelial tissue (254) and at least one sensor capable of detecting at least one of cell morphology and surface topography, the imaging system including at least one of optic scanners, acoustic recognition devices, and photosensor systems.

10. A method for applying an epidermal patch (600), the method comprising the steps of:
locating a position on epidermal tissue (612);
ejecting a quantity of at least one treatment material (618) from an electronically controllable fluid delivery device into contact with the epidermal tissue;
ejecting a quantity of at least one overlayment material (614) into overlying relationship with the epidermal tissue, wherein the overlayment material contains compounds which are essentially inert to epidermal uptake, the overlayment material ejected either contemporaneously or subsequent to ejection of treatment material.

11. A cartridge (800) for use in a device for delivering treatment material to epithelial tissue, the treatment device including at least one scanning device capable of determining at least one of tissue morphology and topography, the device comprising:
a housing (812) removably insertable in the treatment delivery device;
an electronically controllable delivery device (814) contained in the housing;
a reservoir (810) in fluid communication with the fluid delivery device, the reservoir containing at least one fluid treatment material to be applied to epithelial tissue; and
at least one integrated circuit (816) in interactive communication with the treatment delivery device.
